# EUROPEAN PATENT SPECIFICATION

(11) **EP 3 970 716 B1**
(45) Date of publication and mention of the grant of the patent: **25.02.2026**
(21) Application number: 20806509.4
(22) Date of filing: 16.04.2020
(51) Int. Cl.: A61K 31/352, A61P 25/28, A61P 25/16, A23L 33/10, A61K 45/06

(54) **COMPOSITION COMPRISING GOSSYPETIN FOR PREVENTION OR TREATMENT OF ALZHEIMER'S DISEASE**
ZUSAMMENSETZUNG ENTHALTEND GOSSYPETIN ZUR PRÄVENTION ODER BEHANDLUNG DER ALZHEIMER-KRANKHEIT
COMPOSITION COMPRENANT DE LA GOSSYPÉTINE POUR LA PRÉVENTION OU LE TRAITEMENT DE LA MALADIE D'ALZHEIMER

(30) Priority: 14.05.2019 KR 20190056524
(43) Date of publication of application: 23.03.2022
(73) Proprietor: NovMetaPharma Co., Ltd., Gangnam-gu, Seoul (KR)
(72) Inventor: KIM, Kyong Tai, Pohang-si, Gyeongsangbuk-do 37673 (KR); LEE, Dohyun, Pohang-si, Gyeongsangbuk-do 37669 (KR); JO, Kyung Won, Pohang-si, Gyeongsangbuk-do 37673 (KR); JEONG, Younghun, Pohang-si, Gyeongsangbuk-do 37673 (KR)
(74) Representative: Regimbeau
(86) International application number: PCT/KR2020/005080
(87) International publication number: WO 2020/231024

(56) References cited:
- JP-A- 2012 508 740
- JP-A- 2014 198 676
- JP-A- 2014 198 677
- KR-B1- 101 763 315
- US-A1- 2011 111 014
- ZHANG ET AL: "One-compound-multiple-targets strategy to combat Alzheimer's disease", FEBS LETTERS, ELSEVIER, AMSTERDAM, NL, vol. 579, no. 24, 10 October 2005 (2005-10-10), pages 5260 - 5264, XP005098796, ISSN: 0014-5793, DOI: 10.1016/J.FEBSLET.2005.09.006
- TANIGUCHI S ET AL: "Inhibitation of heparin-induced tau filament formation by phenothiazines, polyphenols and porphyrins", JOURNAL OF BIOLOGICAL CHEMISTRY, AMERICAN SOCIETY FOR BIOCHEMISTRY AND MOLECULAR BIOLOGY, US, vol. 280, no. 9, 4 March 2005 (2005-03-04), pages 7614 - 7623, XP003014102, ISSN: 0021-9258, DOI: 10.1074/JBC.M408714200
- C. REMYA, DILEEP K. V., TINTU I., VARIYAR E. J., SADASIVAN C.: "Design of potent inhibitors of acetylcholinesterase using morin as the starting compound", FRONTIERS IN LIFE SCIENCE, vol. 6, no. 3-4, 1 January 2012 (2012-01-01), pages 107 - 117, XP055761078, ISSN: 2155-3769, DOI: 10.1080/21553769.2013.815137

## Description

### [Technical Field]

The present invention relates to a composition containing gossypetin or a salt thereof for the prevention or treatment of an Alzheimer's disease . Furthermore, the present invention relates to a health functional food composition for use in the prevention or alleviation of an Alzheimer's disease, comprising gossypetin or a salt thereof. The present invention also relates to gossypetin or a salt thereof for use for preventing or treating an Alzheimer's disease.

### [Background Art]

The proportion of elderly people is gradually increasing due to the increase of income levels and the development of medical and health environments, especially in Western countries. Korea became an aging society since2017. The proportion of the population who are aged 65 and older exceeded 14% and is expected to become a super-aged society by 2025. Therefore, geriatric diseases of the elderly population are emerging as social problems that must be urgently resolved.

In particular, there are currently neither effective treatments nor prevention methods for Alzheimer's disease, which accounts for about half of senile dementia diseases. Five types of drugs, such as acetylcholine esterase inhibitors and glutamic acid receptor inhibitors, have been approved by the U.S. Food and Drug Administration (FDA) and prescribed to Alzheimer's disease patients, but all of these merely work to relieve symptoms.

Degenerative brain diseases are caused by the deposition of protein aggregates in brain tissues. As for Alzheimer's disease, Aβ peptides aggregate to form plaques outside cells. This results in the generation of reactive oxygen species and the occurrence of an inflammatory response by microglia. IL-1, TNFα, PGE2, NO, NOO⁻, O₂, H₂O₂, and the like are released in microglia to stimulate the apoptosis of surrounding neurons. Reactive oxygen species are generated due to oxidative stress, triggering the release of cytochrome C and activation of caspase-3 in mitochondria and resulting in apoptosis. In addition, with the increase in Aβ peptide aggregation, the aggregation of tau protein, which stabilizes microtubules present in neurons, is also induced. The destruction of microtubules results in the atrophy of neuronal axons and dendrites, causing the degeneration of brain neurons, and the tau protein aggregates accumulate in the cells, causing disorders of neuronal signaling, such as material transport in neurons, resulting in apoptosis.

In addition to Alzheimer's disease, Huntington's disease involves the aggregation of a protein called huntingtin being accumulated in cells, resulting in apoptosis. In particular, when the CAG repeat sequence is genetically generated in the huntingtin gene, 100 or more glutamine residues are attached to the N-terminus of the protein, thereby making it difficult to form a normal protein structure. Moreover, the attenuation of chaperone protein functions makes it difficult to form a three-dimensional protein structure, and misfolded proteins aggregate with one another to form aggregates. Also, in Parkinson's disease, the aggregation of α-synuclein protein is spread to form a filament-like structure, leading to an amyloid fiber structure. Then formation of Lewy bodies increases in cells and inhibits cell functions, ultimately resulting in neuronal apoptosis. The role of normal α-synuclein has recently been revealed. When the concentration of calcium ions in neurons increases, α-synucleinattaches to the endoplasmic reticulum, which stores neurotransmitters, to thereby induce normal secretion of neurotransmitters. The α-synuclein acts as a calcium sensor, and there is a delicate balance between calcium and α-synuclein in cells. Therefore, the disruption of such balance triggers the aggregation of α-synuclein, leading to Parkinson's disease.

As such, the representative symptoms of degenerative brain diseases are caused by increased protein aggregation in brain tissue. The protein aggregation causes an inflammatory immune response in the brain tissue, spreading to surrounding tissues. In addition, intracellular organelle functions decline and apoptotic responses occur, resulting in the degeneration of brain tissue.

Since degenerative brain diseases require the long-term use of drugs, the development of substances with low toxicity and favorable efficacy is essential. Therefore, the development of substances with therapeutic and preventive functions from naturally occurring materials for an edible use can minimize side effects.

Korean Patent No. 10-1424547 and Korean Patent Publication No. 10-2015-0047687 disclose compositions for the treatment of degenerative brain diseases, wherein the compositions contain lactic acid bacteria or products obtained by lactic acid bacterial fermentation, but these are limited in their commercial application due to low treatment effects thereof on degenerative brain disease, such as dementia.

US 2011/111014 A1 methods and compositions for treatment of neurological disorders, in particular Parkinson's disease.

In the literature, Zhang et al. FEBS Lett. 2005, vol. 579, no. 24, (2005-10-10), pp 5260-5264, discloses one-compound-multiple-targets strategy to combat Alzheimer's disease.

In another article, it has also been investigated the inhibitory ability of numerous compounds on the tau filament formation (cf. Taniguchi et al. J. Biol. Chem. 2005, vol. 280, no. 9, pp 7614-7623).

It has also been disclosed a study on the prediction of the acetylcholinesterase (AChE) inhibitory effects of various flavonoid compounds in *silico* (cf. Remya et al. Front. Life Sci., 2012, vol. 6, no. 3-4, pp 107-117).

JP 2012 508740 A relates to a composition and method for treating disorders caused by alpha-synuclein dysfunction and lipid metabolism alteration.

### [Disclosure]

### [Technical Problem]

The present inventors conducted experiments with intensive efforts to complete the present invention by identifying that gossypetin, a small-molecule substance derived from a naturally occurring material, had excellent effects of preventing and treating an Alzheimer's disease.

### [Technical Solution]

An object of the present invention is to provide a pharmaceutical composition containing gossypetin or a salt thereof for the prevention or treatment of an Alzheimer's disease.

Another object of the present invention is to provide a health functional food composition containing gossypetin or a salt thereof for the prevention or alleviation of an Alzheimer's disease.

Still another object of the present invention is to provide gossypetin or a salt thereof for use for preventing or treating an Alzheimer's disease.

### [Advantageous Effects]

It was established in the present invention that gossypetin has an excellent effect of inhibiting protein aggregation in brain cells and also has superb effects of preventing and treating a degenerative brain disease and improving memory in animal experiments, and thus the composition containing gossypetin of the present invention can be helpfully used in the prevention and treatment of an Alzheimer's disease and the improvement of memory and cognitive function.

### [Brief Description of Drawings]

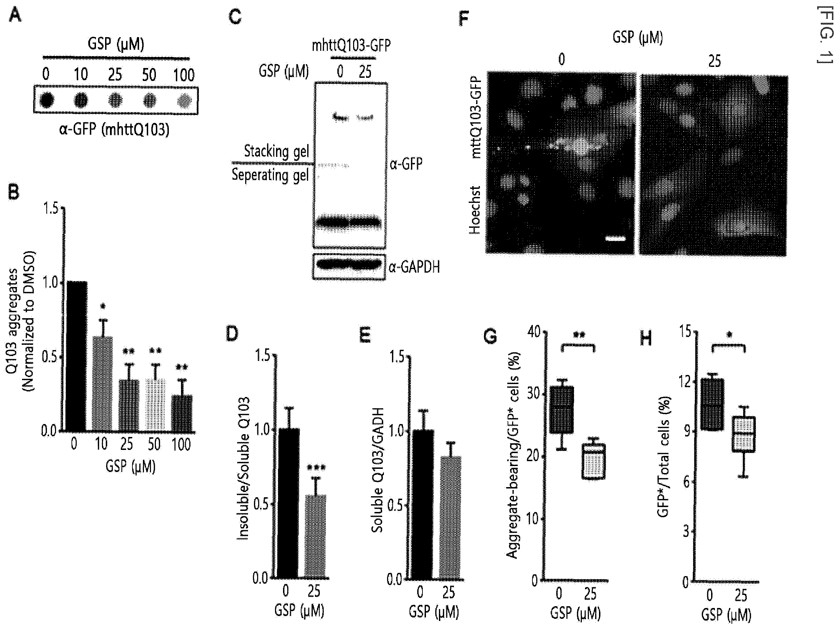

FIG. 1 shows the results depicting an intracellular protein aggregation inhibitory effect of gossypetin. FIG. 1A shows the measurement of the amount of protein aggregates that did not pass through a filter after the treatment of cells with gossypetin, and FIG. 1B shows the amount of protein aggregates confirmed via gel electrophoresis. FIG. 1C shows the formation of protein aggregates and reduction of protein aggregates due to gossypetin treatment in cells, through the aggregation of green fluorescent protein, and FIG. 1D shows quantitative analysis of the results. FIG. 1E shows the comparative analysis of the amount of insoluble protein aggregates relative to soluble protein aggregates, and FIG. 1F shows the analysis of the amount of soluble protein aggregates. FIG. 1G shows the measurement of the number of cells bearing such protein aggregates, and FIG. 1H shows the measurement results of the number of total cells having fluorescent protein expression.
FIG. 2 shows the results depicting the VRK2 activity inhibition according to the concentration of gossypetin.
FIG. 3 shows the results for gossypetin in reassessing the short-term memory of Alzheimer's disease-induced mice through a Y-maze test.
FIG. 4 shows the results for gossypetin in a learning function improvement effect via the measurement of escape latency of Alzheimer's disease-induced mice through a Morris water maze test.
FIG. 5 shows the results for gossypetin in measuring the platform location memory of Alzheimer's disease-induced mice through a Morris water maze test. FIG. 5A shows the measurement of the time of staying at the site where the platform had been located, after the removal of the platform, and FIG. 5B shows the measurement of the total travel path of mice in the water maze test.
FIG. 6 shows the results for gossypetin in measuring the reduction of precipitation of amyloid aggregates in the hippocampus of mouse brain.
FIG. 7 shows the results for gossypetin in measuring the precipitation of amyloid aggregates in the mouse brain. FIG. 7A shows the measurement of the amount of amyloid aggregates that did not pass through a filter among the protein aggregates accumulated in the brain, and FIG. 7B shows the measurement of the amount of soluble amyloid aggregates. FIG. 7C shows the analysis of the amyloid aggregate polymer accumulated in the brain via gel electrophoresis, and FIG. 7D shows the comparative analysis of the amounts of amyloid monomers and polymers.
FIG. 8 shows the results for gossypetin in measuring the reduction in gliosis by microglia in the mouse brain.
FIG. 9 shows the results for gossypetin in measuring the reduction in gliosis by astrocytes in the mouse brain.
FIG. 10 shows the results of comparing the VRK2 activity inhibitory effect of gossypetin with those of compounds known to significantly inhibit the aggregation of Aβ and tau proteins *in vitro.*
FIG. 11 shows the results of comparing the intracellular protein aggregation inhibition of gossypetin with those of compounds known to significantly inhibit the aggregation of Aβ and tau proteins *in vitro.*
FIG. 12 shows the results of comparing, through a Y-maze test, the short-term memory improvement effect in Alzheimer's-induced mouse model due to gossypetin with those due to compounds known to significantly inhibit the aggregation of Aβ and tau proteins *in vitro.*
FIG. 13 shows the results of comparing, through a water maze test, the short-term memory improvement effect in Alzheimer's-induced mouse model due to gossypetin with those due to compounds known to significantly inhibit the aggregation of Aβ and tau proteins *in vitro.*

### [Detailed Description of the Invention]

The present invention will be specifically described as follows. Each description and exemplary embodiment disclosed in this disclosure may also be applied to other descriptions and exemplary embodiments. That is, all combinations of various elements disclosed in this disclosure fall within the scope of the present disclosure. In addition, the scope of the present disclosure is not limited by the specific description below.

Also, a person skilled in the art could recognize or identify numerous equivalents with respect to certain aspects of the present invention using only routine experimentation. Furthermore, such equivalents are intended to be encompassed by the present invention.

An aspect of the present invention provides a pharmaceutical composition containing gossypetin or a salt thereof for the prevention or treatment of an Alzheimer's disease.

The composition contains gossypetin as an active ingredient.

As used herein, the "gossypetin" may be a compound represented by Chemical Formula 1 below:

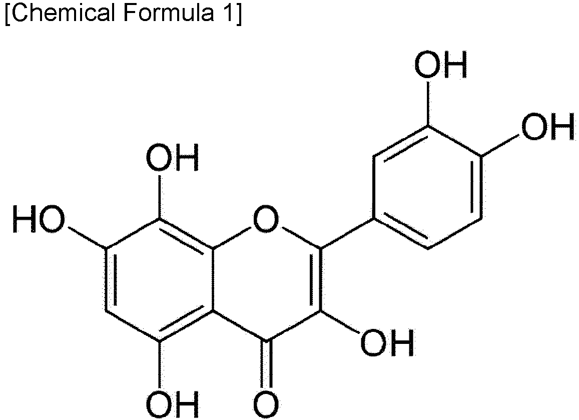

The gossypetin may be a compound named 2-(3,4-dihydroxyphenyl)-3,5,7,8-tetrahydroxy-4H-chromen-4-one.

The gossypetin may be chemically synthesized or extracted from a plant, or it may be a commercially available material, but the method of obtaining gossypetin is not particularly limited and may employ a method known in the art.

The pharmaceutical composition of the present invention may contain not only the gossypetin but also a pharmaceutically acceptable salt thereof. As used herein, the term "pharmaceutically acceptable salt" refers to a salt form in which the compound binds to another substance and means a substance capable of exhibiting pharmaceutical activity similar to that of the compound.

The type of the pharmaceutically acceptable salt includes, but is not limited to, an inorganic acid salt, such as a hydrochloric acid salt, a hydrobromic acid salt, a phosphate salt, or a sulfate salt, and an organic acid salt, such as a carboxylic acid salt or a sulfonic acid salt, but is not limited thereto. The type of the carboxylic acid salt includes an acetic acid salt, a maleic acid salt, a fumaric acid salt, a malic acid salt, a citric acid salt, a tartaric acid salt, a lactic acid salt, or a benzoic acid salt, but is not limited thereto. The type of the sulfonic acid salt includes a methanesulfonic acid salt, an ethanesulfonic acid salt, a benzenesulfonic acid salt, a toluenesulfonic acid salt, or a naphthalenedisulfonic acid salt, but is not limited thereto.

As used herein, the term "degenerative brain disease" refers to a disease caused by brain cell damage.

The degenerative brain disease may be caused by, as a disease cause or a pathological sign, at least one of protein aggregation, increased VRK2 activity, an increased amount of chaperone protein degradation, abnormal protein folding, Lewy body formation, polyglutamine aggregation, and an inflammatory response in neuroglia cells. The protein aggregation may be the aggregation of amyloid Aβ, tau protein, huntingtin, or α-synuclein, but is not limited thereto.

Specifically, the degenerative brain disease of the present invention may be at least one disease selected from the group consisting of dementia, Alzheimer's disease, Huntington's disease, Parkinson's disease, multiple system atrophy, multiple sclerosis, tauopathies, brain tumor, Pick's disease, or Creutzfeldt-Jakob disease. More specifically, the degenerative brain disease of the present invention may be at least one disease selected from Alzheimer's disease, Huntington's disease, and Parkinson's disease, but is not limited thereto.

As used herein, the term "memory/cognitive function" refers to the ability to efficiently manipulate knowledge and information, and encompasses all of the procedures of thinking, speaking, remembering, judging, and executing through the use of the brain. The damage to brain cells may cause the loss or impairment of memory and cognitive function, which may result in symptoms such as forgetfulness, memory decline, and memory disorder.

As used herein, the term "prevention" may refer to any action that suppresses or delays the occurrence of cognitive impairment or neuro-inflammation by administration of the composition according to the present invention. The term "treatment" refers to any action that alleviates or advantageously changes symptoms of an individual with suspected or developed cognitive impairment or neuro-inflammation by way of administration of the pharmaceutical composition. The term "alleviation" refers to any action that at least reduces a parameter associated with a condition to be treated, for example, the severity of a symptom, by way of administration of the composition of the present invention.

It was identified that gossypetin inhibited the VRK2 activity *in vitro,* and the administration of gossypetin to dementia-induced mice improved memory and suppressed the progression of dementia *in vivo.*

The composition of the present invention may further contain an appropriate carrier, excipient, and diluent, which are commonly used to prepare a pharmaceutical composition. In addition, the composition of the present invention may be formulated in the forms of: an oral formulation, such as a powder, granules, a tablet, a capsule, a suspension, an emulsion, a syrup, or an aerosol; an externally applied preparation; a suppository; and a sterile injectable solution, according to the conventional methods.

The pharmaceutical composition containing gossypetin or a pharmaceutically acceptable salt thereof according to the present invention may be formulated in the forms of: an oral formulation, such as a powder, granules, a tablet, a capsule, a suspension, an emulsion, a syrup, or an aerosol; an externally applied preparation; a suppository; and a sterile injectable solution, according to the conventional methods, and may have a formulation of a cream, a gel, a patch, a spray, an ointment, a plaster, a lotion, a liniment, a paste, or a cataplasma, but is not limited thereto.

Examples of the carrier, excipient, and diluent that may be contained in the composition containing gossypetin may include lactose, dextrose, sucrose, oligosaccharides, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oils. The composition, when formulated as a preparation, may be prepared using a diluent or an excipient, such as a filler, an extender, a binder, a wetting agent, a disintegrant, or a surfactant, which is commonly used. Exemplary solid preparations for oral administration include a tablet, a pill, a powder, granules, a capsule, and the like, and such solid preparations may be prepared by mixing the composition with at least one excipient, for example, starch, calcium carbonate, sucrose or lactose, gelatin, or the like. Alternatively, lubricants, such as magnesium stearate and talc, may be used in addition to simple excipients. Exemplary liquid preparations for oral administration correspond to a suspension, a liquid for internal use, an emulsion, a syrup, and the like, and the liquid preparations may contain several types of excipients, such as a wetting agent, a sweetener, a flavor, and a preservative, as well as simple diluents that are frequently used, such as water and liquid paraffin. Exemplary preparations for parenteral administration include a sterile aqueous solution, a non-aqueous solvent, a suspension, an emulsion, a freeze-dried preparation, and a suppository. As the non-aqueous solvent or suspension, propylene glycol, polyethylene glycol, a vegetable oil such as olive oil, an injectable ester such as ethyl oleate, or the like may be used. As a substrate for the suppository, Witepsol, macrogol, Tween 61, cocoa butter, laurin butter, glycerogelatin, or the like may be used.

Another aspect of the present invention provides a health functional food composition containing gossypetin or a salt thereof for the prevention or alleviation of an Alzheimer's disease.

The composition contains gossypetin as an active ingredient.

The gossypetin, degenerative brain disease, memory and cognitive function, prevention, treatment, and alleviation are as described above.

When the gossypetin of the present invention is used as a food additive, the gossypetin may be added as it is, or may be used along with other food or food ingredients, and may be appropriately used by way of a conventional method. The amount of an active ingredient mixed may be appropriately determined according to the purpose of use (prevention, health, or therapeutic treatment). The gossypetin of the present invention is generally added in an amount of 15 wt% or less, preferably 10 wt% or less, relative to raw materials when a food or beverage is manufactured. However, in the case of long-term intake for the purpose of health and hygiene, or for the purpose of regulating health states, the amount may be equal to or less than the above range, and the active ingredient may be used in an amount equal to or greater than the above range since there are no problems in terms of safety.

The type of food is not particularly limited. Examples of the food to which the substance may be added include meat, sausage, bread, chocolate, candies, snacks, confectioneries, pizza, ramen, other noodles, gums, dairy products including ice creams, various soups, beverages, tea, drinks, alcoholic beverages, vitamin complexes, and the like, and encompass all health functional foods in a typical sense.

The health beverage composition according to the present invention may contain various flavors, natural carbohydrates, and the like as additional ingredients, as in typical beverages. The above-described natural carbohydrates may be monosaccharides such as glucose and fructose, disaccharides such as maltose and sucrose, polysaccharides such as dextrin and cyclodextrin, and sugar alcohols such as xylitol, sorbitol, and erythritol. As a sweetener, a natural sweetener such as thaumatin or a stevia extract, a synthetic sweetener such as saccharin or aspartame, or the like may be used. The proportion of the natural carbohydrate is generally about 0.01 g to 0.20 g, and preferably about 0.04 g to 0.10 g per 100 mL of the composition of the present invention.

In addition to the aforementioned ingredients, the composition of the present invention may contain various types of nutrient supplements, vitamins, electrolytes, flavors, colorants, pectic acid and salts thereof, alginic acid and salts thereof, organic acids, protective colloid thickeners, pH adjusters, stabilizers, preservatives, glycerin, alcohols, carbonating agents used in carbonated drinks, and the like. In addition, the composition of the present invention may contain flesh for manufacturing natural fruit juices, fruit juice drinks, and vegetable drinks. These ingredients may be used either alone or in combination. The proportions of these additives do not significantly matter, but are generally selected within a range of 0.01 to 0.20 parts by weight per 100 parts by weight of the composition of the present invention.

Examples of other blended ingredients that may be added may include oil and fat ingredients, a moisturizer, an emollient, a surfactant, organic and inorganic pigments, an organic powder, an ultraviolet absorbent, a preservative, a bactericide, an antioxidant, a plant extract, a pH adjuster, an alcohol, a colorant, a fragrance, a circulation accelerator, a cooling agent, an antiperspirant, purified water, and the like.

Examples of the oil and fat ingredients may include ester-based oils and fats, hydrocarbon-based oils and fats, silicone-based oils and fats, fluorine-based oils and fats, animal oils and fats, plant oils and fats, and the like.

Examples of the ester-based oils and fats may include ester-based oils and fats such as glyceryl tri 2-ethylhexanoate, cetyl 2-ethylhexanoate, isopropyl myristate, butyl myristate, isopropyl palmitate, ethyl stearate, octyl palmitate, isocetyl isostearate, butyl stearate, ethyl linoleate, isopropyl linoleate, ethyl oleate, isocetyl myristate, isostearyl myristate, isostearyl palmitate, octyldodecyl myristate, isocetyl isostearate, diethyl sebacate, diisopropyl adipate, isoalkyl neopentanoate, tri(caprylic, capric acid)glyceryl, tri 2-ethylhexanoic acid trimethylol propane, triisostearic acid trimethylol propane, tetra 2-ethylhexanoic acid pentaerythritol, cetyl caprylate, decyl laurate, hexyl laurate, decyl myristate, myristyl myristate, cetyl myristate, stearyl stearate, decyl oleate, cetyl ricinoleate, isostearyl laurate, isotridecyl myristate, isocetyl palmitate, octyl stearate, isocetyl stearate, isodecyl oleate, octyldodecyl oleate, octyldodecyl linoleate, isopropyl isostearate, 2-ethylhexanoic acid cetostearyl, 2-ethylhexanoic acid stearyl, hexyl isostearate, ethylene glycol dioctanoate, ethylene glycol dioleate, propylene glycol dicaprylate, di(caprylic, capric acid)propylene glycol, propylene glycol dicaprylate, neopentyl glycol dicaprate, neopentyl glycol dioctanoate, glyceryl tricaprylate, glyceryl triundecylate, glyceryl triisopalmitate, glyceryl triisostearate, octyldodecyl neopentanoate, isostearyl octanoate, octyl isononate, hexyldecyl neodecanoate, octyldodecyl neodecanoate, isocetyl isostearate, isostearyl isostearate, octyldecyl isostearate, polyglycerin ester oleate, polyglycerin ester isostearate, isocetyl citrate, triisoalkyl citrate, triisooctyl citrate, lauryl lactate, myristyl lactate, cetyl lactate, octyldecyl lactate, triethyl citrate, acetyltriethyl citrate, acetyltributyl citrate, trioctyl citrate, diisostearyl malate, 2-ethylhexyl hydroxystearate, di-2-ethylhexyl succinate, diisobutyl adipate, diisopropyl sebacate, dioctyl sebacate, cholesteryl stearate, cholesteryl isostearate, cholesteryl hydroxystearate, cholesteryl oleate, dihydrocholesteryl oleate, phytosteryl isostearate, phytosteryl oleate, isocetyl 12-stealoyl hydroxystearate, stearyl 12-stealoyl hydroxystearate, isostearyl 12-stealoyl hydroxystearate, or the like.

Examples of the hydrocarbon-based oils and fats may include hydrocarbon-based oils and fats, such as squalene, liquid paraffin, alpha-olefin oligomers, isoparaffin, sericin, paraffin, liquid isoparaffin, polybutene, microcrystalline wax, and Vaseline, and the like.

Examples of the silicone-based oils and fats may include polymethyl silicone, methylphenyl silicone, methylcyclopolysiloxane, octamethyl polysiloxane, decamethyl polysiloxane, dodecamethyl cyclosiloxane, a dimethylsiloxane-methyl cetyloxysiloxane copolymer, a dimethylsiloxane-methyl stearoxysiloxane copolymer, an alkyl-modified silicone oil, an amino-modified silicone oil, and the like.

Examples of the fluorine-based oils and fats may include perfluoropolyether and the like.

Examples of the animal or plant oils and fats include animal or plant oils and fats, such as avocado oil, almond oil, olive oil, sesame oil, rice bran oil, new flower oil, soybean oil, corn oil, rapeseed oil, apricot kernel oil, palm kernel oil, palm oil, castor oil, sunflower oil, grapeseed oil, cottonseed oil, palm oil, kukui nut oil, wheat germ oil, rice germ oil, shea butter, evening primrose oil, macadamia nut oil, meadow home oil, egg yolk oil, beef tallow, horse oil, mink oil, orange roughy oil, jojoba oil, candelilla wax, carnauba wax, liquid lanolin, and hardened castor oil.

Examples of the moisturizer may include a water-soluble low-molecular-weight moisturizer, a fat-soluble low-molecular-weight moisturizer, a water-soluble polymer, a fat-soluble polymer, and the like.

Examples of the water-soluble low-molecular-weight moisturizer may include serine, glutamine, sorbitol, mannitol, pyrrolidone-sodium carboxylate, glycerin, propylene glycol, 1,3-butylene glycol, ethylene glycol, polyethylene glycol B (polymerization degree n=2 or more), polypropylene glycol (polymerization degree n=2 or more), polyglycerin B (polymerization degree n=2 or more), lactic acid, lactate, and the like.

Examples of the fat-soluble low-molecular-weight moisturizer may include cholesterol, cholesterol ester, and the like.

Examples of the water-soluble polymer may include a carboxyvinyl polymer, polyaspartate, tragacanth, xanthane gum, methyl cellulose, hydroxymethyl cellulose, hydroxyethyl cellulose, hydroxypropyl cellulose, carboxymethyl cellulose, water-soluble chitin, chitosan, dextrin, and the like.

Examples of the fat-soluble polymer may include a polyvinylpyrrolidone-eicosene copolymer, a polyvinylpyrrolidone-hexadecene copolymer, nitrocellulose, dextrin fatty acid ester, high-molecular-weight silicone, and the like.

Examples of the emollient may include long-chain acylglutamate cholesteryl ester, cholesteryl hydroxystearate, 12-hydroxystearic acid, stearic acid, rosin acid, lanolin fatty acid cholesteryl ester, and the like.

Examples of the surfactant may include a nonionic surfactant, an anionic surfactant, a cationic surfactant, an amphoteric surfactant, and the like.

Examples of the nonionic surfactant may include auto-emulsified glycerin monostearate, propylene glycol fatty acid ester, glycerin fatty acid ester, polyglycerin fatty acid ester, sorbitan fatty acid ester, polyoxyethylene (POE) sorbitan fatty acid ester, POE sorbitol fatty acid ester, POE glycerin fatty acid ester, POE alkyl ether, POE fatty acid ester, POE hardened castor oil, POE castor oil, a polyoxyethylene-polyoxypropylene (POE-POP) copolymer, a POE-POP alkyl ether, a polyether-denatured silicone, an alkanolamide laurate, an alkylamine oxide, hydrogenated soybean phospholipid, and the like.

Examples of the anionic surfactant may include fatty acid soaps, α-acylsulfonates, alkyl sulfonates, alkyl allyl sulfonates, alkyl naphthalene sulfonates, alkyl sulfates, POE alkyl ether sulfates, alkylamide sulfates, alkyl phosphates, POE alkyl phosphates, alkylamide phosphates, alkyloyl alkyl taurine salts, N-acylamino acid salts, POE alkyl ether carboxylates, alkyl sulfosuccinates, sodium alkylsulfoacetates, acylated hydrolyzed collagen peptide salts, perfluoroalkyl ester phosphates, and the like.

Examples of the cationic surfactant may include alkyltrimethylammonium chloride, stearyltrimethylammonium chloride, stearyltrimethylammonium bromide, cetostearyltrimethylammonium chloride, distearyldimethylammonium chloride, stearyldimethylbenzylammonium chloride, behenyltrimethylammonium bromide, benzalkonium chloride, diethylaminoethylamide stearate, dimethylaminopropylamide stearate, lanolin derivative quaternary ammonium salts, and the like.

Examples of the amphoteric surfactant may include amphoteric surfactants of the following types: carboxybetaines, amidebetaines, sulfobetaines, hydroxysulfobetaines, amidesulfobetaines, phosphobetaines, aminocarboxylates, imidazoline derivatives and amideamines.

Examples of the organic and inorganic pigments may include: inorganic pigments, such as silicic acid, anhydrous silicic acid, magnesium silicate, talc, sericite, mica, kaolin, Bengala, clay, bentonite, titanium-coated mica, bismuth oxychloride, zirconium oxide, magnesium oxide, zinc oxide, titanium oxide, aluminum oxide, calcium sulfate, barium sulfate, magnesium sulfate, calcium carbonate, magnesium carbonate, iron oxide, ultramarine blue, chromium oxide, chromium hydroxide, calamine, and complexes thereof; organic pigments, such as polyamides, polyesters, polypropylene, polystyrene, polyurethane, vinyl resins, urea resins, phenol resins, fluorine resins, silicone resins, acrylic resins, melamine resins, epoxy resins, polycarbonate resins, a divinyl benzene-styrene copolymer, silk powder, cellulose, CI pigment yellow, and CI pigment orange; complex pigments of these inorganic pigments and organic pigments; and the like.

Examples of the organic powder may include: metallic soaps such as calcium stearate; alkylphosphoric acid metal salts, such as sodium zinc cetylphosphate, zinc laurylphosphate, and calcium laurylphosphate; acylamino acid polyvalent metal salts, such as *N*-lauroyl-β-alanine calcium, *N*-lauroyl-β-alanine zinc, and *N*-lauroylglycine calcium; amidesulfonic acid polyvalent metal salts, such as *N*-lauroyl-taurine calcium and *N*-palmitoyl-taurine calcium; *N*-acyl basic amino acids, such as *N*-ε-lauroyl-L-lysine, *N*-ε-palmitoyl lysine, *N*-α-palmitoyl ornithine, *N-*α-lauroyl arginine, and *N*-α-hardened beef tallow fatty acid acylarginine; *N-*acylpolypeptides, such as *N*-lauroylglycylglycine; α-amino fatty acids, such as α-aminocaprylic acid and α-aminolauric acid; polyethylene; polypropylene; nylon; polymethyl methacrylate; polystyrene; a divinylbenzene-styrene copolymer; ethylene tetrafluoride; and the like.

Examples of the ultraviolet absorbent may include *para*-aminobenzoic acid, ethyl *para*-aminobenzoate, amyl *para*-aminobenzoate, octyl *para*-aminobenzoate, ethylene glycol salicylate, phenyl salicylate, octyl salicylate, benzyl salicylate, butylphenyl salicylate, homomenthyl salicylate, benzyl cinnamate, 2-ethoxyethyl *para*-methoxycinnamate, octyl *para*-methoxycinnamate, glyceryl mono(2-ethylhexanoate) di-*para*-methoxycinnamate, isopropyl *para*-methoxycinnamate, diisopropyl-diisopropyl cinnamic acid ester mixtures, urocanic acid, ethyl urocanate, hydroxymethoxybenzophenone, hydroxymethoxybenzophenonesulfonic acid and salts thereof, dihydroxymethoxybenzophenone, sodium dihydroxymethoxybenzophenonedisulfonate, dihydroxybenzophenone, tetrahydroxybenzophenone, 4-*tert*-butyl-4'-methoxydibenzoyl methane, 2,4,6-trianilino-*p*-(carbo-2'-ethylhexyl-1'-oxy)-1,3,5-triazine, 2-(2-hydroxy-5-methylphenyl)benzotriazole, and the like.

Examples of the bactericide may include hinokitiol, triclosan, trichlorohydroxydiphenyl ether, chlorhexidine gluconate, phenoxyethanol, resorcin, isopropylmethylphenol, azulene, salicylic acid, zinc pyrithione, benzalkonium chloride, photosensitizing dye No. 301, sodium mononitroguaiacol, undecylenic acid, and the like.

Examples of the antioxidant may include butylhydroxyanisole, propyl gallate, erythorbic acid, and the like.

Examples of the pH adjuster may include citric acid, sodium citrate, malic acid, sodium malate, fumaric acid, sodium fumarate, succinic acid, sodium succinate, sodium hydroxide, disodium hydrogen phosphate, and the like.

Examples of the alcohol may include higher alcohols, such as ethyl alcohol.

Further, the blended ingredients that may be added, in addition to the aforementioned ingredients, are not limited thereto, and any of the ingredients can be blended within a range that does not impair the purpose and effect of the present invention, but are blended at preferably 0.01 wt% to 5 wt%, and more preferably 0.01 wt% to 3 wt% relative to the total weight.

When the formulation of the present invention is a lotion, a paste, a cream, or a gel, an animal fiber, a vegetable fiber, a wax, paraffin, starch, tragacanth, a cellulose derivative, polyethylene glycol, silicone, bentonite, silica, talc, zinc oxide, or the like may be used as a carrier ingredient.

When the formulation of the present invention is a powder or a spray, lactose, talc, silica, aluminum hydroxide, calcium silicate, or a polyamide powder may be used as a carrier ingredient, and in particular, when the formulation of the present invention is a spray, the formulation may contain a propellant, such as chlorofluorohydrocarbon, propane/butane, or dimethyl ether.

When the formulation of the present invention is a solution or an emulsion, a solvent, a solubilizer, or an emulsifier is used as a carrier ingredient, and examples thereof include water, ethanol, isopropanol, ethyl carbonate, ethyl acetate, benzyl alcohol, benzyl benzoate, propylene glycol, 1,3-butylglycol oil, glycerol aliphatic esters, polyethylene glycol, or fatty acid esters of sorbitan.

When the formulation of the present invention is a suspension, a liquid diluent, such as water, ethanol, or propylene glycol, a suspension, such as ethoxylated isostearyl alcohol, polyoxyethylene sorbitol ester, or polyoxyethylene sorbitan ester, microcrystalline cellulose, aluminum metahydroxide, bentonite, agar, tragacanth, or the like may be used as a carrier ingredient.

When the formulation of the present invention is a surfactant-containing cleanser, an aliphatic alcohol sulfate, an aliphatic alcohol ether sulfate, sulphosuccinic acid monoester, isethionate, an imidazolinium derivative, methyl taurate, sarcosinate, a fatty acid amide ether sulfate, alkyl amidobetain, an aliphatic alcohol, a fatty acid glyceride, a fatty acid diethanolamide, a vegetable oil, a lanolin derivative, an ethoxylated glycerol fatty acid ester, or the like may be used as a carrier ingredient.

As used herein, the term "individual" refers to a subject in need of the treatment for a disease. Specifically, the individual may refer to a mammal, such as a human or a non-human primate, a mouse, a rat, a dog, a cat, a horse, and a cow, and more specifically an animal excluding a human, or a human, but is not limited thereto.

The pharmaceutical composition according to the present invention may be administered in a pharmaceutically effective amount. The term "pharmaceutically effective amount" refers to an amount sufficient to treat a disease at a reasonable benefit/risk ratio applicable to medical treatment, and the effective dosage level may be determined according to factors including the type of disease in a patient, the severity of disease, the activity of a drug, the sensitivity to a drug, the administration time, the administration route, the excretion rate, the treatment period, and a drug to be co-used, and other factors well known in the medical field. In an embodiment, the concentration of gossypetin administered may be 0.1 mg/kg to 500 mg/kg on the basis of the weight of an individual of administration, and specifically, gossypetin may be administered at a concentration of 0.1 mg/kg to 100 mg/kg, 0.5 mg/kg to 100 mg/kg, 0.5 mg/kg to 50 mg/kg, 0.5 mg/kg to 30 mg/kg, 0.5 mg/kg to 15 mg/kg, or 7 mg/kg to 13 mg/kg, or more specifically 10 mg/kg, but is not limited thereto. The pharmaceutical composition according to the present invention may be administered as an individual therapeutic agent or administered in combination with another therapeutic agent, and may be administered sequentially or simultaneously with a conventional therapeutic agent, and may be administered in either single or multiple doses. It is important to administer the composition in the minimum amount that can exhibit the maximum effect without causing side effects, considering all of the aforementioned factors, and this amount can be easily determined by a person skilled in the art.

The pharmaceutical composition of the present invention may be administered to an individual through various routes. All modes of administration may be contemplated, and for example, administration may be conducted orally or by subcutaneous, intravenous, intramuscular, intrauterine, epidural, or intracerebroventricular injection. The pharmaceutical composition of the present invention is determined according to the type of drug, as an active ingredient, along with various factors, such as a disease to be treated, a route of administration, patient's age, sex, and body weight, and severity of a disease.

### [Mode for Carrying Out the Invention]

Hereinafter, the present invention will be described in detail with reference to examples and experimental examples. However, these examples and experimental examples are provided in order to specifically illustrate the present invention, and the scope of the present invention is not limited thereto.

### Example 1: Identification of in vitro effect of gossypetin

In order to investigate whether gossypetin, a compound derived from a naturally occurring material, had an effect on the suppression of a degenerative brain disease, an *in vitro* experiment was conducted.

### Example 1-1: Identification of concentration-dependent inhibitory effect of gossypetin on intracellular polyglutamine protein aggregation

Polyglutamine protein aggregation is a cause of a degenerative brain disease. In order to investigate the polyglutamine protein aggregation inhibitory effect of gossypetin, U2OS cells expressing VRK2 were treated with different concentrations of gossypetin after the expression of a green fluorescent protein-conjugated polyglutamine protein, and a dot blot assay was performed. The protein aggregates formed in the cells were observed via electrophoresis, and the intracellular protein aggregate formation was observed using a microscope and quantitatively expressed.

It was identified from the results that the polyglutamine protein aggregates were reduced depending on the gossypetin concentration (FIGS. 1A and 1B). In particular, the amount of insoluble protein aggregates was significantly reduced (FIGS. 1C to 1E), and the number of protein aggregate-bearing cells was also decreased (FIGS. 1F to 1H).

### Example 1-2: Identification of interaction between gossypetin (GSP) and VRK2, concentration-dependent inhibition of gossypetin on VRK2 activity, and chaperone protein preservation

The kinase VRK2 is known to prevent the elimination of polyglutamine aggregates by regulating TRiC chaperone protein in cells. Therefore, the inhibition of VRK2 is presumed to suppress the polyglutamine aggregation, and in order to investigate whether gossypetin has a VRK2 activity inhibitory effect, the VRK2 activity inhibition was assayed by treatment with different concentrations of gossypetin.

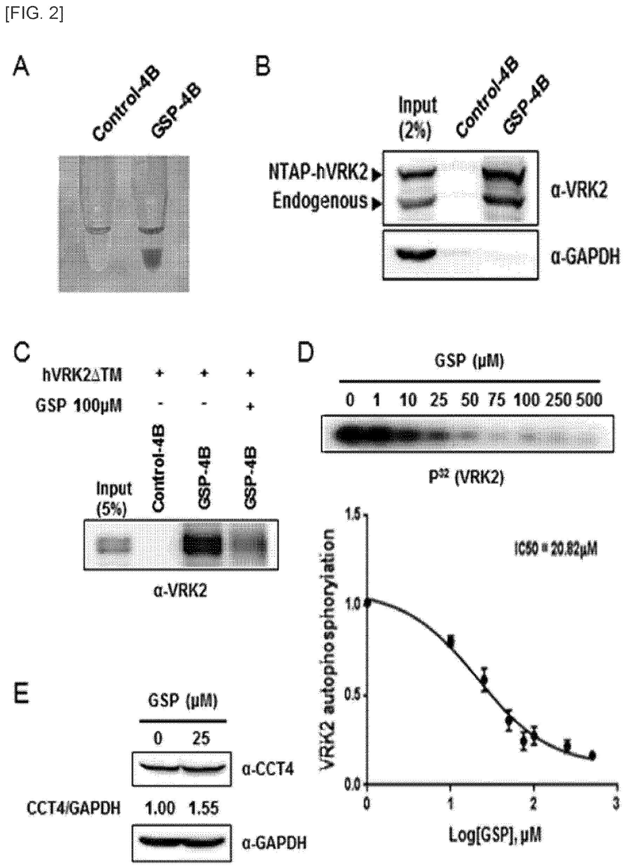

First, gossypetin was chemically bound to Sepharose 4B (FIG. 2A), and then the binding between gossypetin and VRK2 was investigated. VRK2 was identified as binding to only gossypetin-Sepharose 4B without attachment to Sepharose 4B, indicating that VRK2 and gossypetin bound to each other (FIG. 2B). It was also identified that the addition of gossypetin inhibited the binding between VRK2 and gossypetin-Sepharose 4B, revealing that the interaction between of gossypetin and VRK2 was specific (FIG. 2C). The VRK2 activity was inhibited depending on the concentration of gossypetin (FIG. 2D), and the amount of the chaperone protein CCT-4 was not decreased but increased due to the addition of gossypetin (FIG. 2E).

The experimental results identified that gossypetin inhibited VRK2 activity in *vitro,* predicting that gossypetin would have an effect on the prevention and treatment of a degenerative brain disease.

### Example 2: Identification of in vivo effect of gossypetin

In order to investigate whether gossypetin, which was predicted to have an effect on the prevention and treatment of a degenerative brain disease through the results of Example 1, actually has an effect on animals, the animal experiment was conducted.

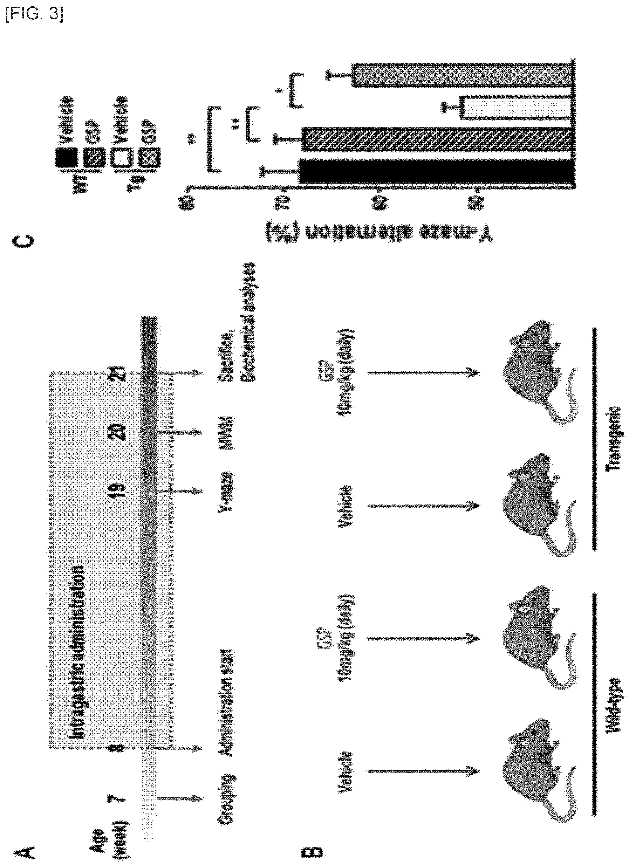

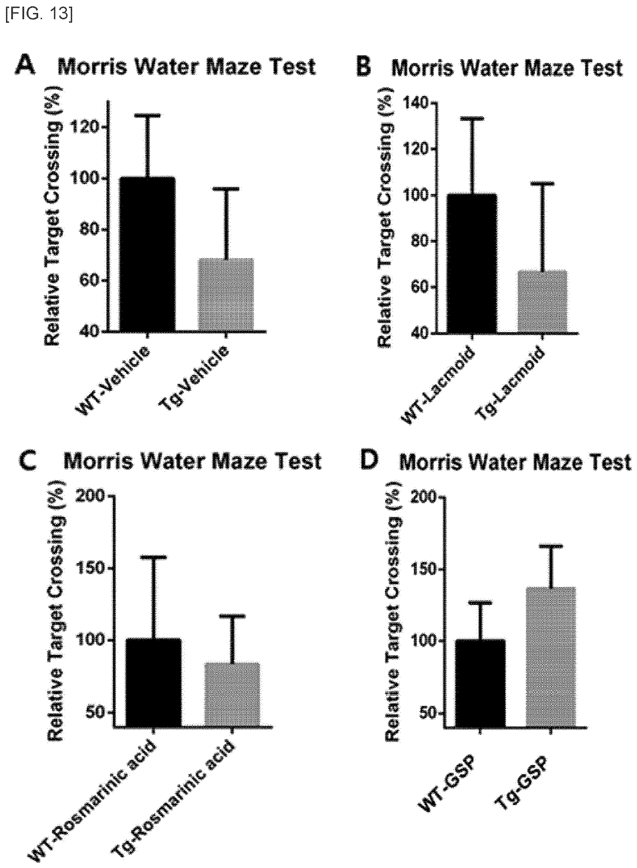

In the experiment, 7-week-old 5xFAD Alzheimer's disease-induced mice were used, and these were orally administered with 10 mg/kg gossypetin for 14 weeks (FIGS. 3A and 3B). Thereafter, Y-maze and Morris water maze tests were performed, which are widely used to check cognitive function. Specifically, short-term memory was assessed through the Y-maze test. The Morris water maze test was performed one week after the Y-maze test. Each mouse was trained to remember the location of the platform for 5 days, and then the time for the mouse to reach the platform was measured. On day 6 after the platform memory training, the platform was removed from the water tank, and the frequency of mouse staying at the site where the platform had been located was measured.

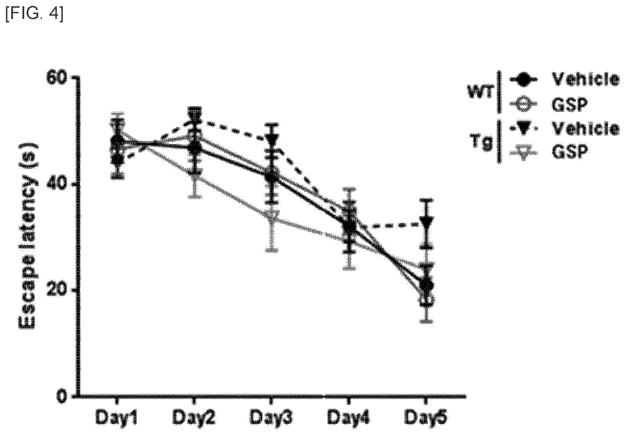

It was identified from the Y-maze test results that the Alzheimer's disease-induced mice 5xFAD administered with gossypetin had improvement in short-term memory to a level similar to that of normal mice (FIG. 3C). As for the results of Morris water maze test, we first investigated whether Alzheimer's disease-induced mice 5xFAD were able to remember the location of the platform through training compared to normal mice. The 5xFAD mice took longer time to find the platform compared to normal mice. However, gossypetin administered 5xFAD group found the platform in a shorter amount of time which was almost as fast as normal mice (FIG. 4).

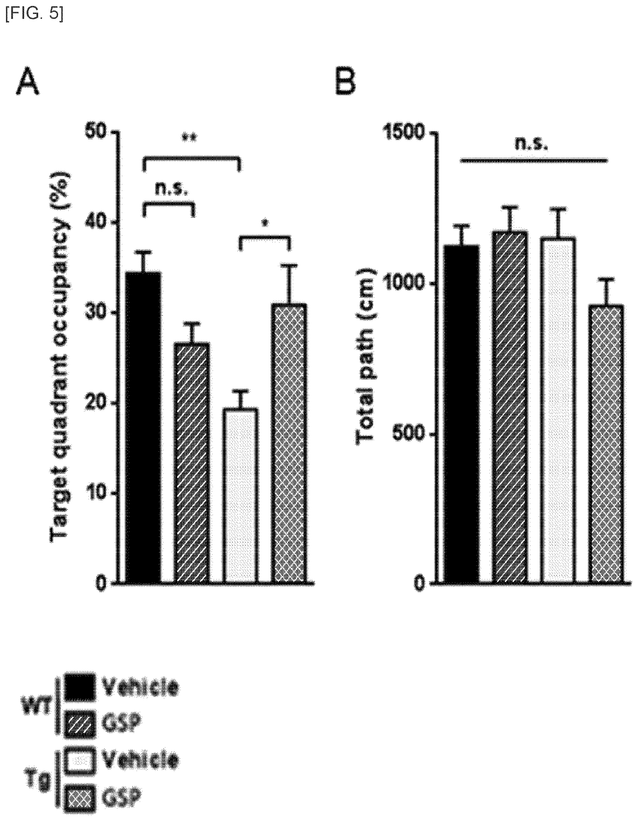

After the platform was removed, the frequency of mice staying at the site where the platform had been located was measured. As a result, the total traveled distance was similar between the groups, but the group of Alzheimer's disease-induced 5xFAD mice fed with gossypetin showed an increased frequency of staying at the site where the platform had been located, indicating that the memory of Alzheimer's-induced mice was improved (FIG. 5).

It was therefore identified that gossypetin had an effect in the prevention and treatment of Alzheimer's disease, and also had an excellent effect in cognitive function improvement.

### Example 3: Analysis of brain tissue of Alzheimer's-induced mice treated with gossypetin

### Example 3-1: Analysis of brain tissue of Alzheimer's-induced mice treated with gossypetin

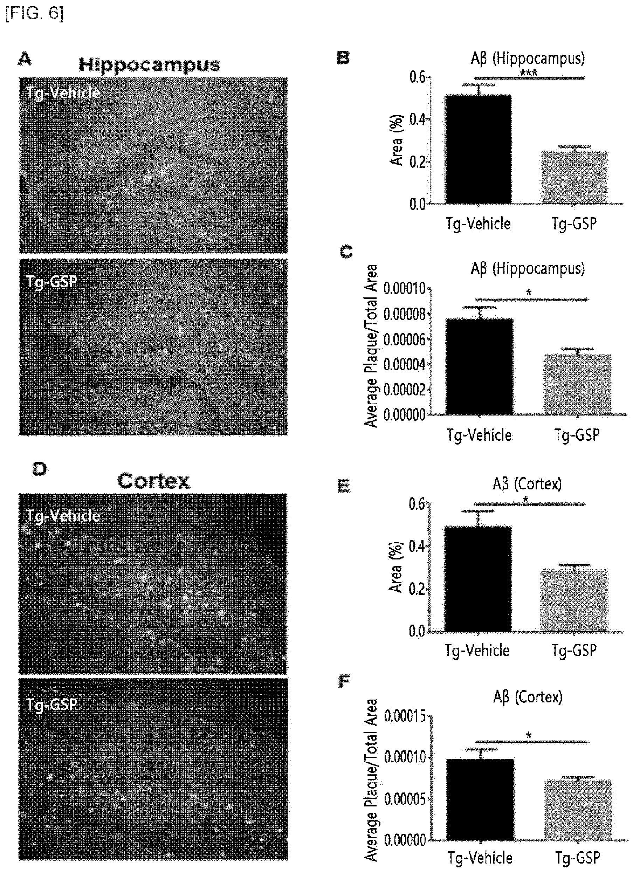

The brains were extracted from the groups of Alzheimer's disease-induced mice fed and not fed with gossypetin and were made into slices, and then the formation of Aβ amyloid aggregates was analyzed using immunohistochemistry staining. In the observation of the hippocampus (FIGS. 6A to 6C) and frontal lobe (FIGS. 6D to 6F) tissues, the Aβ amyloid aggregates were significantly reduced in the group of mice fed with gossypetin.

### Example 3-2: Biochemical analysis of brain hippocampus of Alzheimer's-induced mouse treated with gossypetin

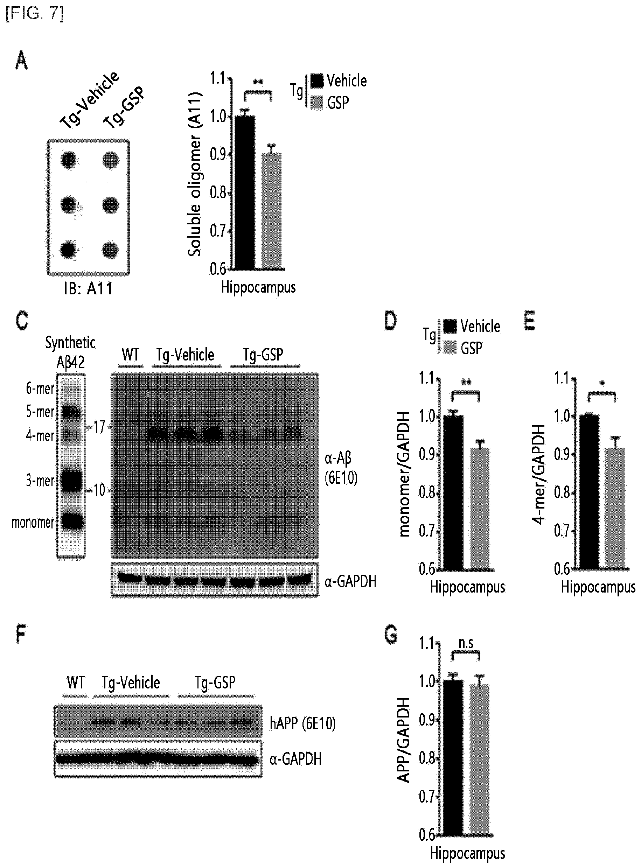

When the brain tissues of Alzheimer's disease-induced mice fed with gossypetin were homogenized and analyzed via biochemical assays, such as protein quantification and western blot, it was identified from the dot blot and western blot results that all of the monomers and polymers as well as soluble Aβ amyloid protein were statistically significantly reduced (FIGS. 7A to 7E). The Aβ amyloid protein is produced by the enzymatic cleavage of the APP amyloid precursor protein. When the western blot was performed to investigate whether the reduction of Aβ amyloid protein resulted from a decrease in the amount of APP amyloid precursor protein, it was identified that the amount of APP amyloid precursor was not changed (FIGS. 7F and 7G).

### Example 3-3: Analysis of gliosis due to hyperactivity of microglia in brain of Alzheimer's-induced mice treated with gossypetin

The brains were extracted from the groups of Alzheimer's disease-induced mice fed and not fed with gossypetin and were made into slices, and then the distribution and proliferation of microglia and astrocytes were analyzed using an antibody against IBA-1, a specific protein of microglia, and an antibody against GFAP, a specific protein of astrocytes.

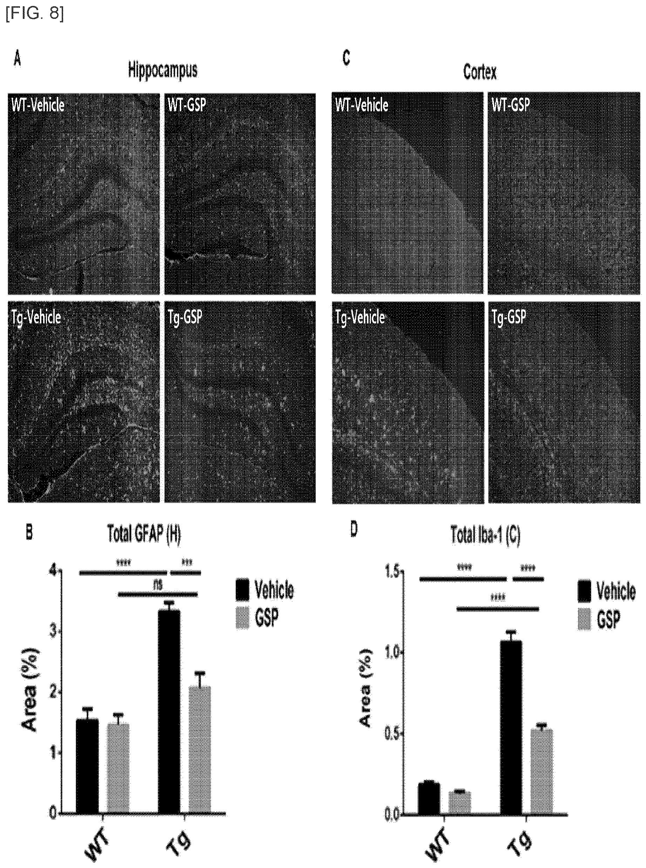

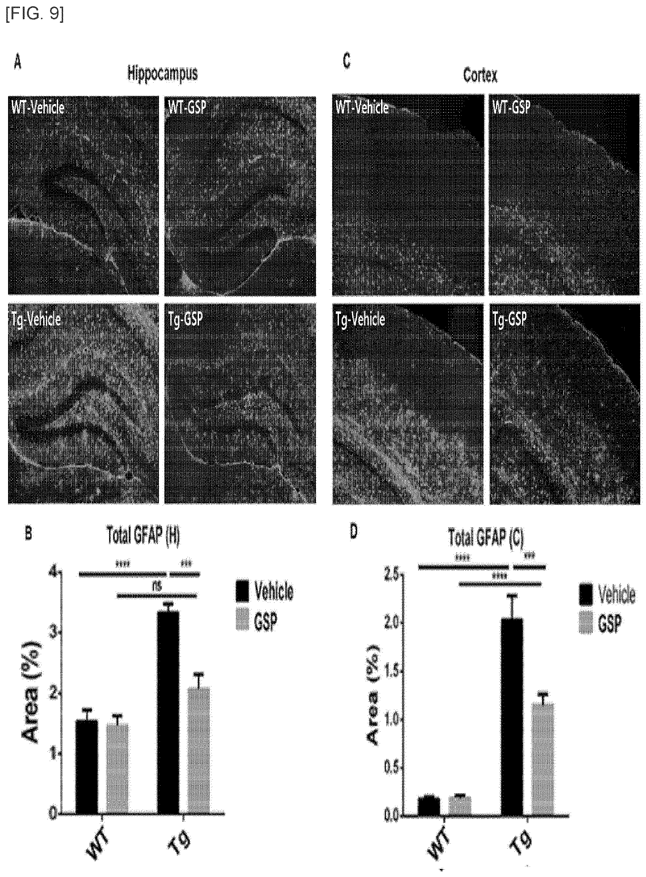

In the observation of the hippocampus (FIGS. 8A and 8B and FIGS. 9A and 9B) and frontal lobe (FIGS. 8C and 8D and FIGS. 9C and 9D) tissues, the hyperactivity of microglia and astrocytes, that is, gliosis, was significantly reduced in the groups of mice fed with gossypetin.

### Example 4: Comparison of gossypetin with other compounds in VRK2 activity inhibitory effect

The VRK2 activity inhibitory level of gossypetin was compared with those of other compounds known to inhibit protein aggregation *in vitro,* which is a cause of a degenerative brain disease.

Specifically, rosmarinic acid and lacmoid, which are known to significantly inhibit the aggregations of Aβ and tau proteins *in vitro,* which are causes of a degenerative brain disease, were measured for VRK2 inhibitory activity via an *in vitro* kinase assay. In addition, cells were treated with each compound together with the induction of polyglutamine protein aggregation, and the reduction in amount of protein aggregation in the cells was observed using a fluorescence microscope. The total area of protein aggregation occurring in the cells, average size of protein aggregation, and inhibition rate of protein aggregation compared with the drug-untreated control group are shown in the table. The cytotoxicity of each compound was also examined.

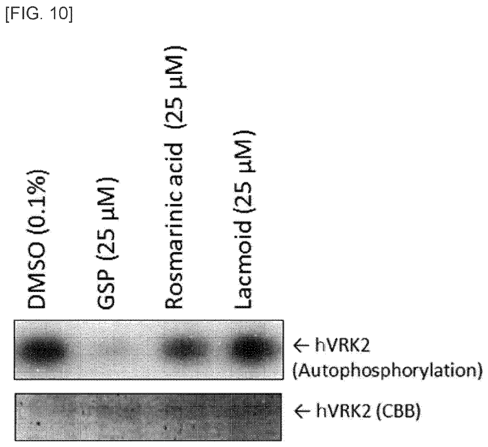

As a result of measuring the autophosphorylation activity of VRK2, at the same concentration of 25 µM, it was shown that gossypetin most strongly inhibited the autophosphorylation activity of VRK2. Rosmarinic acid showed a very weak inhibitory effect, and lacmoid showed insignificant VRK2 activity inhibition (FIG. 10).

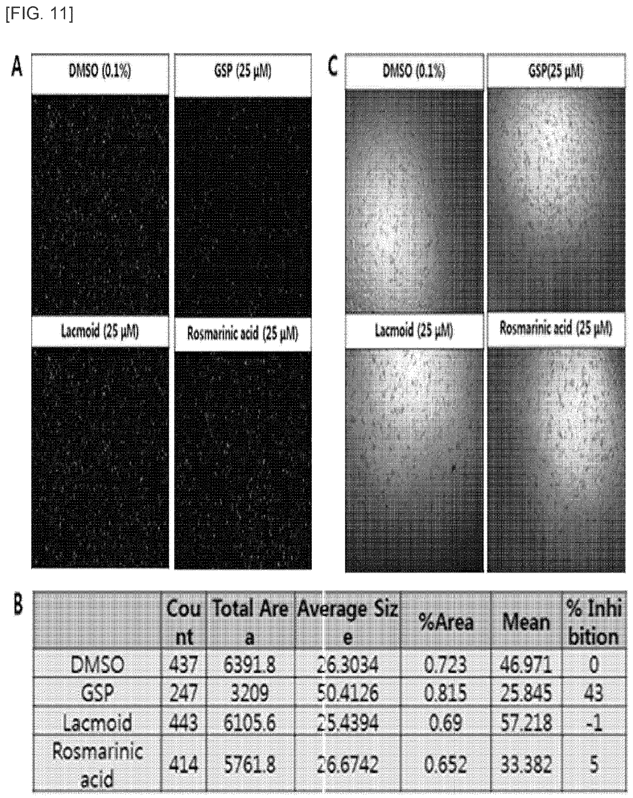

Each of the compounds showed little cytotoxicity (FIG. 11C), and gossypetin showed 43% inhibition of the intracellular protein aggregation, but the other compounds did not inhibit the intracellular protein aggregation (FIGS. 11A and 11B).

It was identified from these results that gossypetin has an excellent protein aggregation inhibitory effect while not showing toxicity in cells. Furthermore, it was identified that gossypetin does not have activity of inhibiting all of the Alzheimer's disease-causing substances despite having the activity of inhibiting some of the Alzheimer's disease-causing substances *in vitro,* leading to the prediction that the effect of gossypetin might be different in the *in vivo* experiment.

### Example 5: Comparison of gossypetin with other compounds in in vivo effect

The compounds in Example 4 were administered to the Alzheimer's disease mouse model to investigate an effect thereof on actual cognitive function improvement.

### Example 5-1: Alzheimer's prevention and treatment effect identified through Y-maze test

Specifically, Alzheimer's disease-induced mouse model 5xFAD was used, and the 5xFAD mice were orally administered 10 mg/kg each of gossypetin (GSP), rosmarinic acid, and lacmoid for 15 weeks from 8 weeks of age, and then the cognitive function was tested via a Y-maze test.

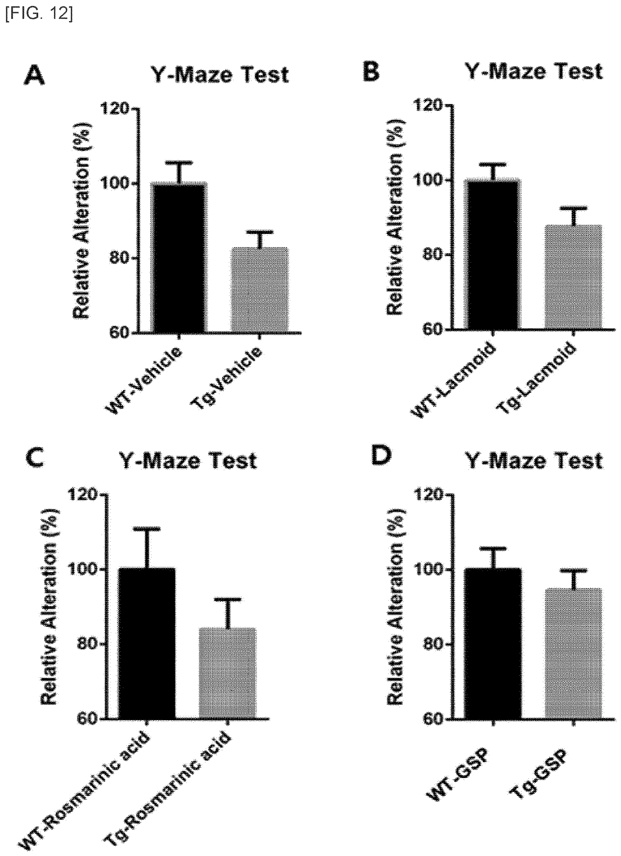

As a test result, the ability of achievement of the 5xFAD mice was significantly diminished compared with normal mice (FIG. 12A), and the treatment with lacmoid (FIG. 12B) or rosmarinic acid (FIG. 12C), compounds known to inhibit the aggregation of Aβ and tau proteins *in vitro,* also did not contribute to the alleviation of such cognitive function decline. However, the administration of gossypetin restored the behavioral alteration to an almost normal level (FIG. 12D), indicating that the cognitive function was improved due to the administration of gossypetin.

### Example 5-2: Alzheimer's prevention and treatment effect identified through water maze test

Specifically, dementia-induced mice 5xFAD were orally administered with 10 mg/kg each of gossypetin (GSP), rosmarinic acid, and lacmoid for 16 weeks from 8 weeks of age, and then the cognitive function was tested via a water maze test. In the water maze test, the mice were trained to find a platform placed under the surface in a water tank for 5 days, and then on Day 6, the platform was removed, and it was monitored how well the mice remembered the site where the platform had been located.

As a test result, the memory of the 5xFAD mice was significantly diminished compared with normal mice (FIG. 13A). The memory was rather defective (FIG. 13B) or was not significantly restored (FIG. 13C) in the 5xFAD mice treated with lacmoid and rosmarinic acid, compounds known to inhibit the aggregation of Aβ and tau proteins *in vitro,* compared to the untreated 5xFAD mice. However, the mice administered with gossypetin showed excellent memory comparable to normal mice (FIG. 13D).

These results identified that even compounds known to have an *in vitro* effect showed different results in an *in vivo* test, and that gossypetin has an excellent effect in cognitive function improvement.

## Claims

1. A pharmaceutical composition for use in the prevention or treatment of an Alzheimer's disease, the pharmaceutical composition comprising gossypetin or a salt thereof.

2. The pharmaceutical composition of claim 1, wherein the gossypetin is represented by Chemical Formula 1 below:

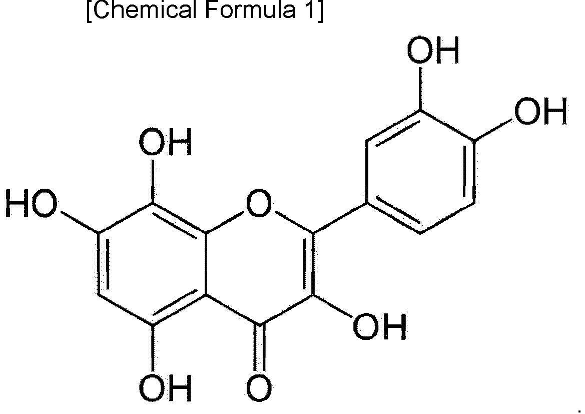

3. The pharmaceutical composition of claim 1, wherein the Alzheimer's disease is caused by at least one cause selected from beta amyloid (Aβ) protein aggregation, and tau protein aggregation.

4. A health functional food composition for use in the prevention or alleviation of an Alzheimer's disease, comprising gossypetin or a salt thereof.

5. Gossypetin or a salt thereof for use for preventing or treating an Alzheimer's disease.

## Patentansprüche

1. Pharmazeutische Zusammensetzung zur Verwendung bei der Prävention oder Behandlung einer Alzheimer-Krankheit, wobei die pharmazeutische Zusammensetzung Gossypetin oder ein Salz davon umfasst.

2. Pharmazeutische Zusammensetzung nach Anspruch 1, wobei das Gossypetin von der folgenden chemischen Formel 1 dargestellt ist:

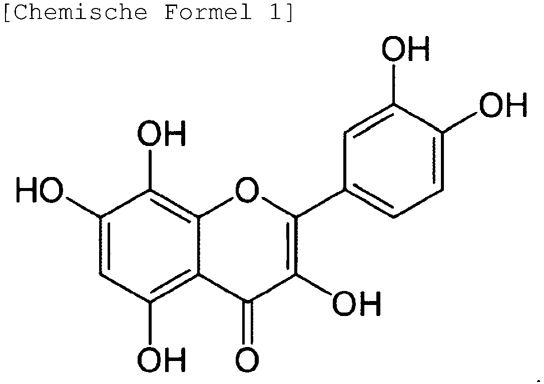

3. Pharmazeutische Zusammensetzung nach Anspruch 1, wobei die Alzheimer-Krankheit von mindestens einer Ursache verursacht wird, die ausgewählt ist aus Beta-Amyloid-(Aß)-Proteinaggregation und Tau-Proteinaggregation.

4. Gesundheitsfördernde Lebensmittelzusammensetzung zur Verwendung bei der Prävention oder Linderung einer Alzheimer-Krankheit, die Gossypetin oder ein Salz davon umfasst.

5. Gossypetin oder ein Salz davon zur Verwendung bei der Prävention oder Behandlung einer Alzheimer-Krankheit.

## Revendications

1. Composition pharmaceutique pour son utilisation dans la prévention ou le traitement de la maladie d'Alzheimer, la composition pharmaceutique comprenant de la gossypétine ou un sel de celle-ci.

2. Composition pharmaceutique selon la revendication 1, dans laquelle la gossypétine est représentée par la formule chimique 1 ci-dessous :

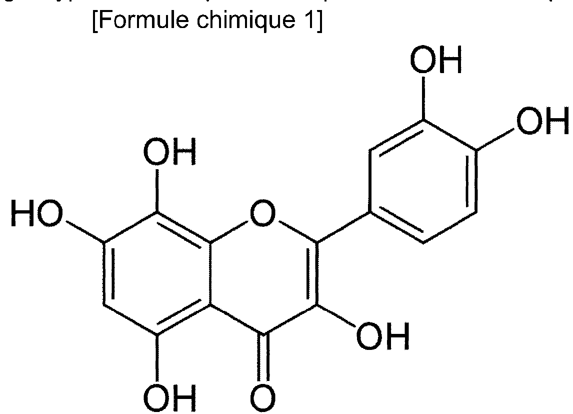

3. Composition pharmaceutique selon la revendication 1, dans laquelle la maladie d'Alzheimer est causée par au moins une cause choisie parmi l'agrégation de la protéine bêta-amyloïde (Aβ) et l'agrégation de la protéine tau.

4. Composition alimentaire fonctionnelle pour la santé pour son utilisation dans la prévention ou le soulagement de la maladie d'Alzheimer, comprenant de la gossypétine ou un sel de celle-ci.

5. Gossypétine ou un sel de celle-ci pour son utilisation dans la prévention ou le traitement de la maladie d'Alzheimer.
